(19) Europäisches Patentamt
European Patent Office
Office européen des brevets

(11) EP 4 279 904 A1

(12) EUROPEAN PATENT APPLICATION

(43) Date of publication:
22.11.2023 Bulletin 2023/47

(21) Application number: 23173727.1

(22) Date of filing: 16.05.2023

(51) International Patent Classification (IPC):
G01N 21/64 (2006.01)   C12Q 1/6818 (2018.01)
C12Q 1/6825 (2018.01)

(52) Cooperative Patent Classification (CPC):
(C-Sets available)
G01N 21/6428; C12Q 1/6818; C12Q 1/6825;
G01N 21/76; G01N 21/6452; G01N 2021/6439

(Cont.)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 16.05.2022  IT 202200010064

(71) Applicant: Optoelettronica Italia S.R.L.
38121 Trento (IT)

(72) Inventors:
• MAGLIONE, Alfredo
38123 Trento (IT)
• DETASSIS, Simone
38121 Trento (IT)
• RESS, Cristina
38122 Trento (IT)
• LUTTERI, Marco
38062 Arco (TN) (IT)

(74) Representative: Caldon, Giuliano et al
Gallo & Partners S.r.l.
Via Rezzonico, 6
35131 Padova (IT)

(54) DEVICE AND METHOD FOR DETETING BIOMOLECULES

(57) Device for detecting biomolecules, comprising a container (2) intended to be filled with a mixture comprising a biomolecule (30) to be detected, a reagent (4) bondable to the biomolecule (30) and susceptible of emitting a first light radiation ($\lambda_1$) in fluorescence or chemiluminescence, and a plurality of magnetic beads (41) chemically bondable to the biomolecule (30). The device comprises a first detector (71) for detecting the first light radiation ($\lambda_1$) for detecting the biomolecule (30) concentration (C), a first emitter (61) for emitting a second light radiation ($\lambda_2$) for irradiating the magnetic beads (41), and a second detector (72) for detecting a third light radiation ($\lambda_3$) consequently transmitted, reflected or emitted by the magnetic beads (41) for detecting the quantity of magnetic beads (41). The device also comprises a logic control unit (8) configured for calculating a correct concentration (C) value of biomolecule (30) on the basis of the above measurements of the light radiations ($\lambda_1$, $\lambda_3$).

Fig. 1

EP 4 279 904 A1

(52) Cooperative Patent Classification (CPC): (Cont.)

C-Sets
**C12Q 1/6818, C12Q 2523/313, C12Q 2537/165,**
**C12Q 2545/114, C12Q 2563/143,**
**C12Q 2563/149, C12Q 2565/607;**
**C12Q 1/6825, C12Q 2523/313, C12Q 2537/165,**
**C12Q 2545/114, C12Q 2563/143,**
**C12Q 2563/149, C12Q 2565/101**

**Description**

Field of application

The present invention relates to a device and a method for detecting biomolecules, according to the preamble of the respective independent claims.

The device and method in question are intended to be advantageously used to detect, or identify and quantify, a biomolecule, in particular a nucleic acid, in a liquid or solid sample.

In more detail, the device and method object of the present patent are used in the diagnostic, prognostic, predictive and pharmaceutical fields, in order to perform an accurate, reproducible, preferably highly automated analysis capable of detecting very low biomolecule concentrations, for example of the order of picomolar.

The device object of the present invention therefore fits into the industrial sector of the production of biomedical and pharmaceutical analysis devices.

Background art

In recent years, research in the diagnostic and pharmaceutical fields has required increasingly sophisticated devices and analysis methods in order to identify biomolecules of interest, for example in order to be able to implement an early diagnosis, implement a more accurate diagnosis and to develop new drugs and therapeutic targets.

It has been known for some time that specific biomolecules, such as for example gene sequences of nucleic acids, are associated with specific information relating to the state of health of a patient, his/her response to a drug, or are used for therapeutic purposes. In particular, therapies based on natural or chemically modified oligonucleotides, for example ASO (antisense oligonucleotides), have proved to be of particular relevance in the pharmacological approach. The aforesaid oligonucleotides are molecules of different sizes, from tens to hundreds of base pairs, and allow hybridization via complementary sequence of other nucleic acids to increase, modify or decrease the expression thereof and/or modify the base sequence thereof. Furthermore, nucleic acids, including for example microRNAs (miRNAs), have been studied as possible biomarkers of physiological or pathological states allowing diagnostic, prognostic and predictive analyses.

In general, for the purpose of developing new drugs, the identification and quantification of these nucleic acids for therapeutic purposes is rather complicated, as the chemical modifications made make it difficult to analyze them with standard methods such as PCR (polymerase chain reaction). Likewise, the identification and quantification of nucleic acids for diagnostic, prognostic and predictive purposes is equally complicated, as the concentration of these molecules is particularly low and standard methods such as PCR are not very reproducible.

Currently, current detection techniques exploit methods based on optical principles, such as for example FRET (Förster Resonance Energy Transfer) analysis, which involves exploiting the energy transfer between two fluorescent molecules at a suitable distance. In general, only in the presence of the nucleic acid under test is a luminous intensity proportional to the concentration of the nucleic acid itself generated.

Devices are known on the market which are able to carry out analyses of biomolecules, in particular nucleic acids, or portions of the latter.

Generally, these devices comprise a container, provided with a plurality of containment cavities, into which a mixture to be analyzed is introduced. This mixture comprises in particular: the actual sample which contains the biomolecule to be detected, a reagent, in particular comprising the marker capable of emitting a luminous intensity, and a plurality of magnetic beads, which act as a support to immobilize the biomolecules of interest and to which probe molecules capable of hybridizing with the biomolecule (i.e. in particular the nucleic acid) to be detected are generally bonded (by means of a chemical bond).

Generally, the number of probe molecules bonded to each magnetic bead, and therefore of biomolecules that may bind to them, is given by a probability distribution, for example a Poisson distribution.

After the components of the mixture are placed in the containment cavities and once the reaction between them has taken place, it is obtained that the biomolecules to be detected are each linked to a corresponding probe molecule and, directly or indirectly, to a marker to form a complex supported by the magnetic bead.

Generally, the aforesaid devices also comprise means for emitting a luminous radiation capable of emitting an excitation radiation at a frequency such as to excite the marker and cause the latter to emit a fluorescence radiation, different from the excitation radiation. In particular, the fluorescence radiation will be all the more intense the greater the quantity of marker and therefore of biomolecule to which it is bound.

The device further comprises a sensor arranged for detecting the fluorescence radiation and generating a signal representative of the quantity of nucleic acid within the sample.

The device further comprises a logic control unit, which receives the input signal from the sensors and, on the basis of this signal, calculates the quantity of biomolecule present in the sample.

The devices of the known type described above have proved to be not free from drawbacks in practice.

A first drawback is given by the fact that these devices are not very precise. In particular, between the introduction of the sample into the container and the actuation of the device to carry out the detection, at least one washing is performed to remove the unreacted sample portion. During the washing, the magnetic beads are immobilized by a magnetic field to avoid the loss of biomolecule to be detected and however even this operation is not able to ensure that the quantity of magnetic beads remains the same as the starting one.

Consequently, since the number of magnetic beads (and therefore also the quantity of biomolecule bound to them) removed is indefinite and varies on the basis, for example, of the reaction conditions, the measurement loses precision.

A further drawback is given by the fact that, for the same reasons indicated above, these devices are not very accurate. Indeed, sample dilution during washing and loss of magnetic beads results in a variable decrease in biomolecule concentration. Furthermore, both magnetic beads bound to biomolecules and magnetic beads not bound to biomolecules are accidentally eliminated from the container in quantities which are not known in the two cases and which are always variable. Therefore, the uncertainty of these quantities makes the result obtained difficult to repeat.

A further drawback is given by the fact that most of the methods for detecting biomolecules, in particular methods using the aforesaid devices, are affected by random errors due to the operator who carries out the analyses. In fact, these methods provide for numerous manual steps, in which the intervention of the operator is required, which may introduce possible errors in the dosages of sample, reagent and washing liquid, and possible contamination of the sample to be analyzed.

Alternatively, in case the analysis is of the fully automated type, often the sensor or the containment cavity are moved to different operating positions during the analysis and the alignment between the containment cavity, the sensor and the emission means takes some time of setting that may compromise the reproducibility of the analysis.

Disclosure of the invention

Under this situation, the problem underlying the present invention is that of providing a device and a method for detecting biomolecules capable of providing a highly accurate result.

Another object of the present invention is to provide a device and a method for detecting biomolecules capable of providing a highly precise result.

A further object of the present invention is to provide a device and a method for detecting biomolecules capable of employing several analytical techniques on the same sample.

A further object of the present invention is to provide a device and a method for detecting biomolecules, which provide repeatable and reproducible results.

A further object of the present invention is to provide a device and a method for detecting biomolecules, which are easy and quick to implement.

A further object of the present invention is to provide a device for detecting biomolecules which do not require the movement of the sample to be analyzed with respect to the various components of the device itself.

Brief description of the drawings

The technical features of the invention, according to the aforesaid aims, may clearly be seen in the content of the claims below, and its advantages will become more readily apparent in the detailed description that follows, made with reference to the accompanying drawings, which illustrate a preferred embodiment, which is purely exemplary and not limiting, in which:

- Figure 1 shows a schematic representation of the device for detecting biomolecules object of the invention;
- Figure 2 shows a detail of the device in Figure 1, relating to a container of a mixture to be analyzed, according to the present invention;
- Figure 3A and Figure 3B show schematic representations of a detail of the device of Figure 1, relating to illumination means and to a detection module, according to a first and a second embodiment of the present invention;
- Figure 4 shows a detail of the second embodiment of the device of Figure 1, relating to a detection module incorporating an emitter of the illumination means;
- Figures 5A and 5B show a detail of two variants of a third embodiment of the device of Figure 1, relating to a detection module;
- Figure 6 shows a schematic representation of a chemiluminescent reaction between a reagent and a biomolecule contained in a sample to be analyzed by means of the device of Figure 1;
- Figure 7 shows a schematic representation of a fluorescence reaction between a reagent and a biomolecule contained in a sample to be analyzed by means of the device of Figure 1, with a fluorophore highlighted.

Detailed description of a preferred embodiment example

With reference to the accompanying drawings, reference number 1 indicates as a whole the device for detecting biomolecules object of the present invention.

The device 1 may advantageously be used in the biomedical and/or pharmaceutical field for detecting biomolecules in a biological sample, such as blood, urine or tissue.

In particular, the term "biomolecules" shall mean nucleic acids, proteins or peptides. Advantageously, the device is particularly suitable for the detection of nucleic acids, such as for example RNA, DNA, PNA, miRNA, mRNA, siRNA, ASO, etc.

According to the invention, the device 1 for detecting biomolecules comprises a container 2 provide3d with at least one containment cavity 20.

The containment cavity 20 delimits a containment volume intended to be at least partially filled with a mixture to be analyzed.

Preferably, the container 2 is provided with a plurality of containment cavities 20, each delimiting a corresponding containment volume intended to be at least partially filled with the mixture to be analyzed.

Advantageously, the container 2 is provided, on one side thereof, with a detection window 21 which is transparent at least to a first light radiation $\lambda_1$ and to a third light radiation $\lambda_3$, and advantageously also to a second light radiation $\lambda_2$, which will be described in more detail below. Preferably, the detection window 21 is optically transparent to all light radiations at least in the visible, infrared and ultraviolet ranges.

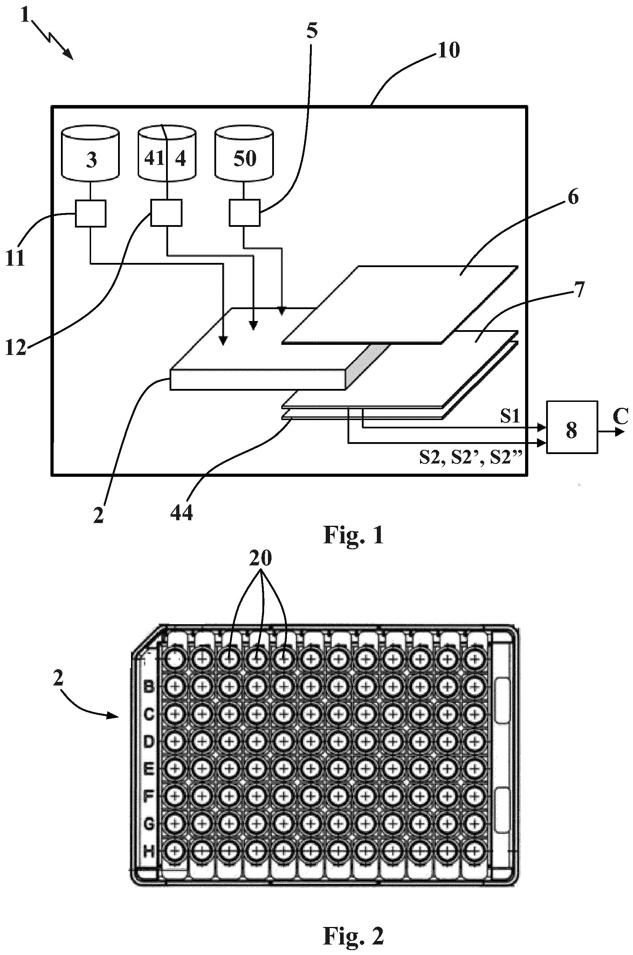

Preferably, the container 2 is a microtitration plate of a type known and standardized in the sector, for example of the type illustrated in Figure 2. In particular, the microtitration plate is rectangular or circular in shape, and is preferably made of advantageously transparent plastic material. Furthermore, the containment cavities 20 are preferably cylindrical, or of the type called in technical jargon "wells", and in numbers equal to 6, 12, 24, 48, 96, 384 or 1536, according to ANSI standards.

Alternatively, the container 2 may comprise a plurality of test tubes or cuvettes, which define the aforementioned containment cavities 20 and are adapted to contain the mixture to be analyzed.

Advantageously, the test tubes or cuvettes are made of transparent material, for example quartz, in particular a material which is transparent to electromagnetic radiation in the visible, UV, far-UV (FUV), near-UV (NUV), and infrared ranges (IR) to allow irradiation of the mixture to be analyzed.

In such cases, the detection window 21 may be formed for example at the bottom of the microtitration plate, test tubes or cuvettes.

According to the invention, the mixture to be analyzed comprises a sample 3 susceptible of containing at least one biomolecule 30 to be detected, such as for example, as explained above, nucleic acids, or sequences of nucleic acids, or, alternatively, proteins or peptides.

Furthermore, the mixture to be analyzed comprises a reagent 4, that may be bonded to the biomolecule 30 and susceptible of emitting a first light radiation $\lambda_1$ in fluorescence or chemiluminescence, and a plurality of magnetic beads 41, wherein each magnetic bead 41 is chemically bondable, preferably bonded, to the biomolecule 30.

The term "reagent" will obviously mean a single substance, or one or more mixtures of different substances (possibly to be metered at different times of the preparation process of the mixture to be analyzed) advantageously capable of reacting, bonding together and/or to the biomolecule 30, and of emitting the first light radiation $\lambda_1$ in fluorescence or chemiluminescence.

Hereinafter, the expression magnetic beads will mean objects defined in the technical jargon of the sector as "beads". In particular, such magnetic beads are generally beads with a diameter of the order of a few tens of nanometers up to about 100 $\mu$m, made of super paramagnetic material, for example ferrite, which exhibits magnetic behavior only in the presence of an external magnetic field.

Optionally, the magnetic beads 41 are provided with a fluorescent compound, i.e. capable of emitting fluorescent light radiation if suitably excited with another light radiation as better described hereinafter.

According to the embodiments of the invention, the magnetic beads 41 preferably comprise a polymeric matrix and paramagnetic nanoparticles incorporated within the polymeric matrix.

In the case of fluorescent magnetic beads 41, the fluorescent compound is preferably incorporated within said polymeric matrix. In particular, the fluorescent compound comprises Europium and/or Terbium and/or cyanines. Preferably, the fluorescent compound comprises Europium.

For example, 41 magnetic beads called FG-beads or FF-beads manufactured by the Tamagawa Seiki company may be used.

Advantageously, the mixture to be analyzed further comprises at least one probe molecule 42 which is chemically bonded to the magnetic beads 41. In particular, the probe molecule 42 is complementary or similar to the biomolecule 30 to be detected in the sample 3, and the biomolecule 30 is advantageously bonded to the magnetic beads 41 via the probe molecule 42, preferably by hybridization. For example, in the case where the biomolecule 30 to be detected is ASO, the probe molecule 42 is preferably a peptide nucleic acid (PNA), the nucleotide bases of which are synthesized in such a way that they may bind in a complementary way to the ASO molecule to be detected.

Advantageously, each magnetic bead 41 is bound to several probe molecules 42, and the number of probe molecules 42 bound to a magnetic bead 41 follows a Poisson distribution in a known manner.

Advantageously, the average value of the number of probe molecules 42 of the distribution varies in relation to the size ratio between the magnetic bead 41 and the probe molecule 42. In particular, as known, as the steric bulk of a probe molecule 42 increases, the number of probe molecules adjacent to the latter per unit area decreases.

Advantageously, the containment volume of the containment cavities 20 is between 1 $\mu$L and 4 mL, preferably about 50 $\mu$L, and is preferably capable of being filled between half and two thirds of the mixture to be analyzed, so as to allow the possible introduction of other liquids inside the containment cavities 20, for example washing liquids, as described in more detail below.

Moreover, the device comprises at least one detection module 7, associable with the containment cavity 20 (or with at least one of the containment cavities 20) of the container 2 and comprising at least one first detector 71, which is arranged for detecting the first light radiation $\lambda_1$ from the containment cavity 20 in order to generate a first signal S1 representative of the concentration C of biomolecule 30 within the containment cavity 20.

In particular, the intensity of the first signal S1 is directly proportional to the intensity of the first light radiation $\lambda_1$ and advantageously to the concentration of the biomolecule 30 present in sample 3.

In particular, the first detector 71 comprises at least one first sensor 710. Furthermore, the first detector 71 comprises at least one first optical filter 74' provided with a first spectral band corresponding to the first light radiation $\lambda_1$.

According to the idea underlying the present invention, the device 1 further comprises illumination means 6 comprising at least one first emitter 61 arranged for emitting at least one second light radiation $\lambda_2$ within the containment volume of the containment cavity 20 in order to irradiate the magnetic beads 41. In particular, the magnetic beads 41 are susceptible of at least partially absorbing the second light radiation $\lambda_2$ and consequently transmitting, reflecting or emitting a third light radiation $\lambda_3$ associated with the quantity of magnetic beads 41 in the containment cavity 20.

More in detail, in the case in which the second light radiation $\lambda_2$ is transmitted or reflected, the third light radiation $\lambda_3$ has the same wavelength as the second light radiation $\lambda_2$ but a lower intensity, i.e. it is a fraction of the second light radiation $\lambda_2$, depending on the quantity of magnetic beads 41. Conversely, if the magnetic beads 41 are provided with a fluorescent compound, as indicated above, the magnetic beads 41 themselves are advantageously capable of absorbing at least part of the second light radiation $\lambda_2$ and emitting the third light radiation $\lambda_3$ with a different wavelength and intensity substantially proportional to the quantity of magnetic beads 41.

Preferably, the first emitter 61 comprises an LED, which is susceptible of emitting the second light radiation $\lambda_2$, preferably at a wavelength between 400 and 450 nm, preferably about 414 nm. Furthermore, the first emitter 61 preferably comprises an attenuator, which is arranged for reducing the intensity of the second light radiation $\lambda_2$ emitted.

The detection module 7 comprises a second detector 72, arranged for detecting at least the third light radiation $\lambda_3$ coming from the magnetic beads 41 contained in the containment cavity 20 and generating a second signal S2 representative of the quantity of magnetic beads 41 within the container 2. In particular, the second detector 72 comprises at least one second sensor 720. Furthermore, the second detector 72 advantageously comprises at least one second optical filter 74" provided with a second spectral band corresponding to the third light radiation $\lambda_3$ and different from the first spectral band. In particular, the second optical filter 74" is arranged for blocking the detection of light radiations having a wavelength different from the wavelength range of the second light radiation $\lambda_2$ and/or of the third light radiation $\lambda_3$.

Advantageously, the first detector 71 and the second detector 72 are arranged for being positioned optically coupled to the same detection window 21.

According to the preferred embodiment of the invention, the detection module 7 comprises an integrated circuit 75. Furthermore, advantageously, the first sensor 710 of the first detector 71 and the second sensor 720 of the second detector 72 are mounted on the same integrated circuit 75.

In this way, it is advantageously possible to detect the concentration of biomolecule 30 and the concentration of magnetic beads 41 without the need to move the sample with respect to the detection module 7, reducing the possibility of alignment errors between the detection module 7 and the container 2 and increasing the overall reproducibility of the concentration measurement C of the biomolecule 30.

Advantageously, the detection module 7 also comprises a support base 70 on which the integrated circuit 75 is mounted on which at least the first sensor 710 and the second detector 720 are mounted. Furthermore, advantageously, the first optical filter 74' and the second optical filter 74" are fixed to the support base 70 to cover, respectively, the first sensor 710 and the second sensor 720.

Advantageously, in order to reduce the mutual interference phenomena between the optical filters 74, the detection module 7 comprises one or more optically opaque walls 76 interposed at least between the first optical filter 74' and the second optical filter 74". For example, these optically opaque walls 76 may be the side walls themselves of the optical filters 74 covered by an optically opaque coating (for example black), or alternatively, the optically opaque dividing partitions arranged between the different optical filters 74.

The device 1 further comprises a logic control unit 8 in data communication with the first and second detectors 71, 72 and configured for receiving the first signal S1 and the second signal S2 and calculating, on the basis of the first signal S1 and the second signal S2, a correct value of concentration C of biomolecule 30 within the containment cavity 20.

In this way, the device 1 allows the concentration C of biomolecule 30 to be measured with accuracy and repeatability in relation to the number of magnetic beads 41 contained in the mixture at the moment of detection.

Preferably, a washing liquid 50 may also be inserted and subsequently removed in the containment volume of the containment cavity 20 to remove at least a discard portion of the sample 3 and/or unreacted reagent 4.

Advantageously, the first emitter 61 is arranged for emitting at least the second light radiation $\lambda_2$ inside the containment volume and the second detector 72, arranged for detecting the third light radiation $\lambda_3$ coming from the magnetic beads 41 in at least two instants of time successive to each other. In particular, a first time $t_1$ is prior to the introduction of the washing liquid 50 into the containment cavity 20 and a second time $t_2$ is subsequent to the removal of the washing liquid 50 from the containment cavity 20. In this case, advantageously, the second detector 72 is arranged for generating two corresponding second signals S2', S2" and the logic control unit 8 is configured for calculating the correct value of concentration C of biomolecule 30 on the basis of both second signals S2', S2" (as well as on the basis of the first signal S1).

In this way, advantageously, the calculation of the concentration C of biomolecule 30 also takes into account the number of magnetic beads 41 lost during the washing.

In particular, according to a first non-limiting embodiment of the present invention, the logic control unit 8 advantageously calculates the corrected concentration C of biomolecule 30 starting from the second signal S2, according to Formula 1 shown below:

$$S1 = A_1 \cdot C + A_2 \cdot S2'' + A_3 + A_4 \cdot C^2 + A_5 \cdot S2''^2 \qquad (1)$$

wherein $A_{1-5}$ are coefficients advantageously calculated and considered on the basis of the bonding efficiency between the biomolecule 30 and the magnetic beads 41, or more particularly on the basis of the hybridization efficiency between the probe molecule 42 and the biomolecule 30.

Furthermore, the signal S2' is advantageously used (if detected) to validate the correctness of the aforementioned coefficients $A_{1-5}$.

Alternatively, according to a second embodiment example, the logic control unit 8 advantageously calculates the corrected concentration C of biomolecule 30 starting from the second signal S2, according to Formula 2 shown below:

$$S1 = A_1 \cdot C + A_2 \cdot S2'' + A_3 \qquad (2)$$

wherein $A_{1-3}$ are coefficients advantageously calculated and considered on the basis of the bonding efficiency between the biomolecule 30 and the magnetic beads 41, or more particularly on the basis of the hybridization efficiency between the probe molecule 42 and the biomolecule 30.

Furthermore, the signal S2' is advantageously used (if detected) to validate the correctness of the aforementioned coefficients $A_{1-3}$.

Advantageously, the above formulas may conveniently include other terms of the quadratic type or of higher degree, depending on the experimental features of the measurement.

Therefore, more generally, according to the non-limiting embodiments of the present invention shown above, the logic control unit 8 advantageously calculates the corrected concentration C of biomolecule 30 starting from the second signal S2, according to Formula 1':

$$S1 = A_0 + \sum_n (A_{2n-1} \cdot C^n + A_{2n} \cdot S2''^n) \qquad (1')$$

wherein n is a natural number greater than or equal to 1, $A_{0,2n-1,2n}$ are coefficients advantageously calculated and considered on the basis of the bonding efficiency between the biomolecule 30 and the magnetic beads 41, or more particularly on the basis of the hybridization efficiency between the probe molecule 42 and the biomolecule 30.

Preferably, one or more of the coefficients $A_{0,1,2,3,4,5,2n-1,2n}$, preferably all, are calculated empirically. In particular, the aforementioned coefficients are determined by regression (for example linear or quadratic regression) using an empirical curve which relates the relationship between the first signal S1 relating to the detected concentration of biomolecule 30 and the second signal S2 relating to the detected concentration of magnetic beads 41, and the ratio between known concentrations of biomolecule 30 and magnetic beads 41 (and consequently of probe molecules 42 anchored to the magnetic beads 41).

In this way, it is possible to associate different coefficients with corresponding probe molecules 42, increasing the specificity of the analysis on the basis of the probe molecule 42 used.

Furthermore, the signal S2' is advantageously used (if detected) to validate the correctness of the aforementioned coefficients $A_{0,2n-1,2n}$.

According to the first and second embodiments, the correct concentration value C is calculated by inverting the formulas and isolating such unknown.

For example, in the second embodiment, Formula 3 is obtained:

$$C = (S1 - A_2 S2'' - A_3) / A1 \qquad (3)$$

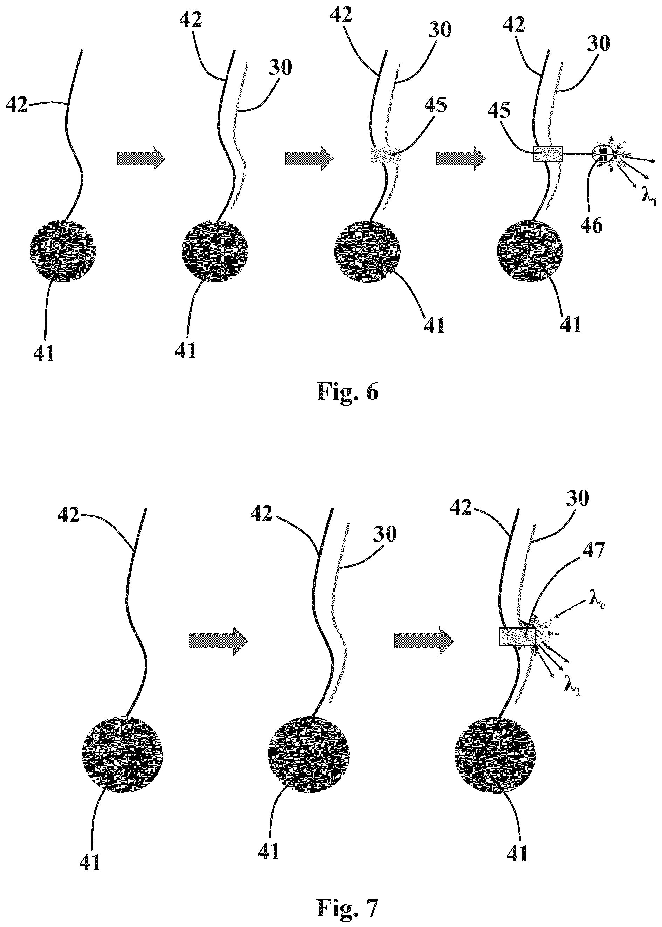

According to a first detection method of the present invention, schematically illustrated in Figure 7, the detection of the biomolecule is performed by means of a fluorescence technique. In this case, the device 1 is arranged for performing an analysis of the optical type in fluorescence, for example an analysis of the FRET type.

Advantageously, according to the first detection method, the reagent 4 comprises a fluorophore 47 bondable to the biomolecule 30 and susceptible of emitting the first light radiation $\lambda_1$ in fluorescence. The fluorophore 47 may therefore advantageously be associated with the hybridization or bonding event between the probe molecule 42 and the biomolecule 30.

In particular, the fluorophore 47 is a recognizable molecule capable of being optically excitable. This recognizable molecule is advantageously bonded directly to the probe molecule 42 or, alternatively, is associated with the probe molecule 42 at a later time (for example following the successful hybridization/bond between the probe molecule 42 and the biomolecule 30) in such a way that, when irradiated by an excitation radiation $\lambda_e$, it is able to emit the first light radiation $\lambda_1$. In particular, if the fluorescence analysis is of the FRET type, the reagent comprises a first fluorophore 47' and a second fluorophore 47", wherein the first fluorophore 47' is susceptible of being excited by the excitation radiation $\lambda_e$ and it emits an auxiliary electromagnetic radiation able to excite the second fluorophore 47", which in turn emits the first light radiation $\lambda_1$.

Furthermore, again in the case of a FRET analysis, the first detector 71 is advantageously provided with two first sensors 710, one of which is arranged for detecting the auxiliary electromagnetic radiation emitted by the first fluorophore 47' and the other sensor to detect the first light radiation $\lambda_1$ emitted by the second fluorophore 47".

An example of fluorophore 47 is reported in detail in patent EP 2195459. This patent describes a kit comprising modified nucleic bases bonded to an optically identifiable molecule, i.e. to a fluorophore 47, which is for example fluorescein, 5-dimethylaminonaphthalene-1-sulfonyl chloride (dansyl) or other fluorescent molecules known in the field.

Advantageously, the illumination means 6 comprise at least one second emitter 62, arranged for emitting at least one light excitation radiation $\lambda_e$ within the containment volume of the containment cavity 20 in order to irradiate the fluorophore 47 and cause the emission of the first light radiation $\lambda_1$ in fluorescence.

Preferably, the second emitter 62 comprises an LED, which emits the excitation light radiation $\lambda_e$, preferably at a wavelength between 320 and 380 nm, preferably about 365 nm. Furthermore, the second emitter 62 comprises an optical emission filter, preferably of the 390 - SP type, which lets through the wavelengths between 325 and 370 nm.

More in detail, the first detector 71 is arranged for detecting the first light radiation $\lambda_1$ in fluorescence, preferably light radiations in the visible spectrum. For this purpose, the first sensor 710 of the first detector is arranged for detecting the first light radiation $\lambda_1$ in fluorescence and the first optical filter 74' is suitably selected on the basis of the fluorophore 47 used.

Advantageously, the first optical filter 74' of the first detector 71 is a filter capable of filtering a specific range of wavelengths in the visible range, such range extends around the wavelength of the first light radiation $\lambda_1$ emitted.

According to a second detection method of the present invention, schematically illustrated in Figure 6, the detection of the biomolecule is performed by means of a chemiluminescence technique. In this case, the device 1 is arranged for performing an analysis of the optical type in chemiluminescence.

Advantageously, the reagent 4 comprises a first molecule 45 susceptible of reacting at least with the biomolecule 30 and optionally with a second molecule 46 bonded to the biomolecule 30 in order to emit the first light radiation $\lambda_1$ in chemiluminescence.

In particular, the reagent 4 comprises the first molecule 45 and the second molecule 46. The first molecule 45 is advantageously bondable to the probe molecule 42 hybridized or associated with the biomolecule 30, and to the second molecule 46 to emit the first light radiation $\lambda_1$ in chemiluminescence.

Of course, the reagent 4 may optionally be devoid of the second molecule 46, and the first molecule may be bonded to the probe molecule 42 hybridized or associated with the biomolecule 30 to emit the first light radiation $\lambda_1$.

Furthermore, the first detector 71 is adapted to detect the first light radiation $\lambda_1$ in chemiluminescence.

For this purpose, the first sensor 710 is adapted to detect NUV-visible light radiation (i.e. in the near ultraviolet and visible range) and the first optical filter 74' is suitably selected on the basis of the reagent 4 used.

According to the aforementioned second detection mode, the second emitter 62 is not present or is not operative, since the reaction itself generates the first light radiation $\lambda_1$.

Advantageously, the first and/or second sensor 710, 720 are selected from a SiPM (silicon photomultiplier) sensor and a SPAD (single photon photodetector diode) sensor.

Advantageously, the detection module 7 of the device 1 comprises two first detectors 71, one of which is arranged to detect the first light radiation $\lambda_1$ in fluorescence and one to detect the first light radiation $\lambda_1$ in chemiluminescence, and may therefore advantageously carry out, depending on the need and on the preparation of the mixture containing the sample 3, only one or both types of detection, according to the first and/or the second detection method.

According to the preferred embodiment of the invention, the illumination means 6 advantageously comprise at least one third emitter 63, which is arranged for emitting at least one control light radiation $\lambda_c$ within the containment volume of the containment cavity 20 to irradiate at least the sample 3.

Furthermore, the detection module 7 is provided with a third detector 73, which is arranged for detecting a fraction of the control radiation $\lambda_c$ from the containment cavity 20 and for generating a third signal $S_3$ representative of the qualitative state, in particular of absence or presence of hemolysis of the sample 3 within the containment cavity 20.

In particular, the detection of the third detector 73 is performed in transmission mode, i.e. the containment cavity 20 is advantageously placed between the third detector 73 and the third emitter 63.

Advantageously, the third emitter 63 coincides with the first emitter 61 and emits a control radiation $\lambda_c$, preferably between 400 and 440 nm, even more preferably of about 414 nm. Furthermore, the third detector 73 is advantageously provided with a third sensor 730, for example of the SPAD or SiPM type, and preferably with a third optical filter 74''', in particular calibrated around the wavelength of the control radiation $\lambda_c$. Preferably, the third sensor 730 is mounted on the same integrated circuit as the detection module 7.

In this way, the arrangement of the third detector 73 and of the third emitter 63 advantageously allows detecting in the sample 3, in the case the sample 3 consists mainly of blood, the phenomenon of haemolysis, i.e. the rupture of red blood cells, in a preliminary manner with respect to the analysis of the concentration C of biomolecule 30, thus excluding the reading of the possibly hemolyzed sample 3. As known, haemolysis in fact prevents obtaining a reliable detection of the concentration C of biomolecule 30, as it decreases the qualitative state of sample 3.

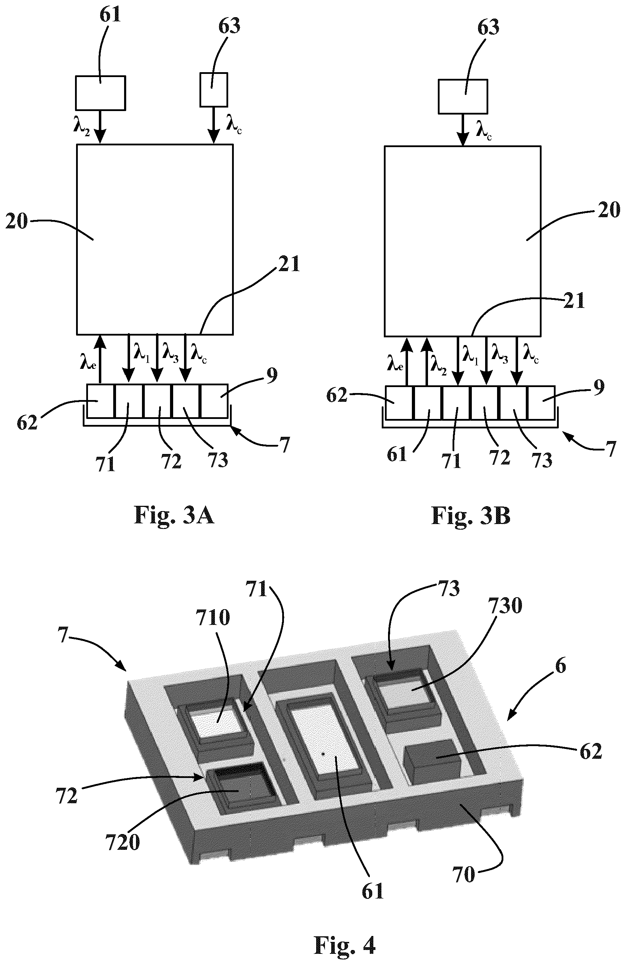

According to a first embodiment, illustrated in Figure 3A, the container 2 is removably arranged with its containment cavity 20 (or its containment cavities 20) interposed between the first emitter 61 of the illumination means 6 and the detection module 7.

In this way, it is advantageously possible to carry out, by means of the second detector 72, a measurement in transmittance, wherein the concentration of magnetic beads 41 may be identified for example by means of Lambert Beer's law, according to Formula 4 shown below:

$$M = A/(\varepsilon \cdot l) \tag{4}$$

where M is the concentration of the magnetic beads 41, *A* is the absorbance (calculated as the opposite of the natural logarithm of the measured transmittance), $\varepsilon$ is the molar absorption coefficient of the magnetic beads 41 and *l* is the geometric path traversed by the light radiation $\lambda_2$.

Preferably, the geometric path *l*, also known as the "optical path", is between 5 and 10 mm, so as to allow the detection module 7 to receive most of the second light radiation $\lambda_2$ and the third light radiation $\lambda_3$.

Furthermore, by minimizing the geometric path *l*, it is possible to avoid the use of deflectors and/or dichroic mirrors.

According to a second embodiment of the present invention, illustrated in Figure 3B, the first emitter 61 of the illumination means 6 is integrated in the detection module 7 and the detection module 7 is placed above or below the container 2. In particular, the second detector 72 of the detection module 7 is placed close to the first emitter 61.

In this way, the concentration of the magnetic beads 41 may be measured in reflectance. In this case, operatively, the second light radiation $\lambda_2$ emitted by the first emitter 61 enters the containment cavity 20, reaches the magnetic beads 41 arranged preferably on the bottom of the latter and is reflected in various directions. The third light radiation $\lambda_3$, i.e. the fraction of the second light radiation $\lambda_2$ redirected towards the detection module 7, in particular towards the second detector 72, is then detected by the latter.

Preferably, the second emitter 62 is integrated in the detection module 7, as also illustrated in Figure 4. In particular, Figure 4 shows an example of a detector according to the second embodiment of the invention, in which the first detector 71, the second detector 72 and the third detector 73, the first emitter 61 and the second emitter 62 are mounted on a single integrated circuit.

According to embodiment variants of the first and second embodiments, in the case in which the magnetic beads 41 comprise a fluorescent compound, as anticipated above, the first emitter 61 is advantageously susceptible of emitting the second light radiation $\lambda_2$ in fluorescence, for example with a wavelength between 320 and 380 nm, preferably about 365 nm, thus acting like the excitation radiation $\lambda_e$.

In this case, the first emitter 61 advantageously also comprises at least one optical filter capable of filtering a specific range of wavelengths, in particular in the wavelength range indicated above.

In this case, the container 2 is advantageously removably arranged with its containment cavity 20 (or its containment cavities 20) interposed between the first emitter 61 of the illumination means 6 and the detection module 7 (similarly to the first embodiment in Figure 3A), or the first emitter 61 of the illumination means 6 is advantageously integrated in the detection module 7 and the detection module 7 is placed above or below the container 2 (similarly to the second embodiment described above and in Figure 3B).

Operatively, following the irradiation of the magnetic beads 41 with the aforementioned radiation, the latter (in particular their fluorescent compound) are susceptible of emitting the third light radiation $\lambda_3$, detectable by the second detector 72. Of course, the magnetic beads 41 may also either transmit or reflect a fraction of the second light radiation $\lambda_2$, or re-emit the third light radiation $\lambda_3$, and both may be detected by the second detector 72.

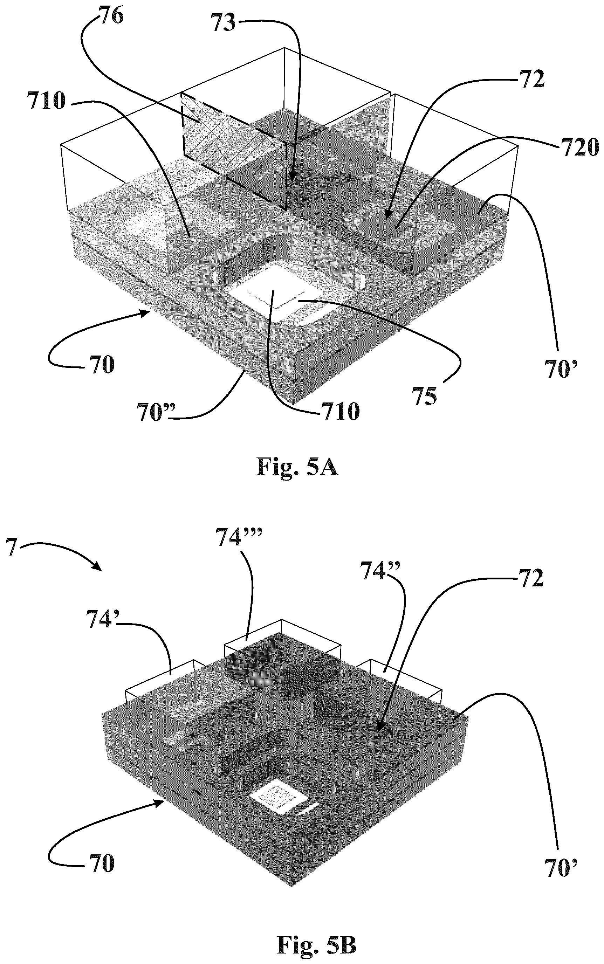

As anticipated above, according to a third embodiment illustrated in Figures 5A and 5B, the detection module 7 comprises a support base 70, made for example of polymeric material, on which the integrated circuit on which at least the first detector 71 and the second detector 72 are mounted, is advantageously mounted.

Advantageously, the support base 70 extends between a first main upper face 70' and an opposite second main lower face 70". In particular, the support base 70 has a parallelepiped shape.

Furthermore, the support base 70 is advantageously provided with at least two housing seats, preferably at least four housing seats. Advantageously, each housing seat is obtained starting from an upper opening, made on the first main face 70', in proximity of which a portion of the container 2 is intended to be placed, and a bottom wall, on which one between the first, second and third sensors 710, 720, 730 respectively of the first, second or third detectors 71, 72, 73 is fixed.

Advantageously, the integrated circuit 75 puts at least the first detector 71 and the second detector 72 into data communication with the logic control unit 8.

Preferably, the aforementioned sensors 710, 720, 730 are SiPM sensors, with an area comprised between 0.25 mm$^2$ and 1 mm$^2$. Advantageously, the reduced area of the aforementioned sensors 710, 720, 730 allows the detection module 7 to be placed directly optically coupled to the detection window 21 of the container 2, without the need to move one between the container 2 and the detection module 7 during analysis by the device.

In more detail, if the support base 70 is provided with four seats, the latter are arranged according to a 2x2 matrix.

Advantageously, as illustrated in Figure 5A, each containment seat extends between the upper opening and the bottom wall with a substantially constant section.

Advantageously, furthermore, as visible in Figure 5A, each optical filter 74', 74", 74''' of the detectors 71, 72, 73 of the detection module 7 is fixed to the support base 70, in particular to the first main face 70' of the latter, for example by gluing, to cover the corresponding sensor 710, 720, 730. In more detail, each optical filter 74', 74", 74''' is placed to cover the upper opening of the housing seat in which the corresponding sensor 710, 720, 730 is housed. In this way, each optical filter 74', 74", 74''' may be used to let pass at the same time a corresponding wavelength detectable by the underlying sensor 710, 720, 730, making the acquisition of the signals S1, S2', S2" faster.

In particular, each optical filter 74', 74", 74''' has a parallelepiped shape and is placed between the detection window 21 of the container 2 and the support base 70 of the detection module 7.

Alternatively, it is possible to advantageously use an optical filter in a single body divided into a number of sections equal to that of the sensors 710, 720, 730 mounted on the support base 70. These sections are advantageously distinguished by applying coatings with different filtering properties on the different areas of the optical filter in a single body, each corresponding to an opening of a specific housing seat of the support base 70.

According to an embodiment variant illustrated in Figure 5B, each housing seat extends between the upper opening and the bottom wall with a variable section. In particular, the inner side walls of the housing seat define a shoulder which causes a narrowing of the section at an intermediate point between the upper opening and the bottom wall.

In this way, each optical filter 74', 74", 74''' associated with each sensor 710, 720, 730 of the detection module 7 is inserted at least partially within the corresponding housing seat, in particular in such a way that it rests on the shoulder. Preferably, each optical filter 74', 74", 74''' is fixed, for example by gluing, to the corresponding shoulder.

In this way, the optical filters 74', 74", 74''' are kept separate from each other, advantageously decreasing the mutual interference phenomena.

Advantageously, according to the third embodiment illustrated in Figures 5A and 5B, the mutual interference phenomena between the various optical filters 74', 74", 74''' are further avoided, for example by covering the walls of the various optical filters 74', 74", 74''' with an optically opaque coating (i.e. black in color), or alternatively by applying optically opaque dividing partitions (i.e. black in color) between the various optical filters 74', 74", 74'".

According to a first example of the third embodiment, the detection module 7 is provided with two first optical filters 74' associated with corresponding first sensors 710 for respectively isolating the first light radiation $\lambda_1$ in fluorescence and the first light radiation $\lambda_1$ in chemiluminescence, a second optical filter 74" associated with the second sensor 720 of the second detector 72 for isolating the third light radiation $\lambda_3$ and a third optical filter 74''' associated with a third sensor 730 of the third detector 73 for isolating the control light radiation $\lambda_c$.

According to a second example, the detection module 7 comprises four first sensors 710 and four respective first optical filters 74', two of which are arranged for isolating the first light radiation $\lambda_1$ in fluorescence and two arranged for isolating the first light radiation $\lambda_1$ in chemiluminescence. In this way, it is possible to perform a double acquisition of the first light radiation $\lambda_1$, alternatively in fluorescence or chemiluminescence according to the need, and to perform an average of the first signals S1 acquired by each first sensor 710, and therefore obtain a more accurate result.

Of course, without thereby departing from the scope of protection of the present invention, different combinations of the embodiments described above are possible. For example, the first and second embodiments may also include detectors 71, 72, 73 constructed as in the third embodiment and in which at most the first and/or second emitters 61, 62 are integrated.

In this case, in particular, the support base 70 carries mounted the first emitter 61 and/or the second emitter 62 arranged for example laterally on two opposite sides of the support base 70.

Preferably, each emitter 61, 62 is mounted on a heat dissipating element, for example a Peltier plate, such dissipating element is advantageously mechanically fixed in turn to the support base 70. In this way, it is avoided to excessively heat the sensors 710, 720, 730 and the sample contained in the container 2 and consequently compromise the determination of the concentration value C determined by the logic control unit 8.

The device 1 advantageously comprises at least one temperature sensor 9, associable with at least one between the container 2 and the detection module 7 in order to detect a temperature value and generate at least one temperature signal relative to the temperature value.

Advantageously, the logic control unit 8 is configured for receiving the temperature signal and verifying that the measurement occurs in conditions of stability and correct operation of the detection module 7.

Optionally, in combination or alternatively, the logic control unit 8 is configured for calculating the correct concentration value C of biomolecule 30 inside the container 2 also on the basis of the temperature signal.

In this way, it is possible to obtain a precise and accurate measurement even in the event of temperature variations in the external environment and inside the device 1.

In particular, for the same quantity of light radiation which strikes the detection module 7, in the event of a temperature increase, the logic control unit 8 is configured for advantageously calculating a concentration value C at an established standard temperature. In this way, it is possible to correct any measurement errors due to the sensitivity of the sensors to temperature variations, especially for temperatures above 30 °C.

Furthermore, the first detector 71, the second detector 72 and/or the third detector 73 are preferably coupled at least in part to a Peltier cell, which is arranged for regulating the temperature of the sensors, advantageously minimizing the correction to be applied by means of the temperature signal.

According to the preferred embodiment, the device 1 comprises an external structure 10, which internally defines an operating volume and is made of material capable of insulating the operating volume from external light sources. Furthermore, the external structure 10 is advantageously provided with at least one access opening through which it is possible to introduce the container 2 into the operating volume. This allows more precise qualitative and quantitative analyses of an optical type to be performed, without the result being influenced by the light coming from the external environment.

Advantageously, the device 1 is arranged for performing simultaneous detections on samples 3 arranged in different containment cavities 20 of the container 2, if the latter is provided with them.

To this purpose, the illumination means 6 comprise a plurality of first emitters 61, each associated with a corresponding containment cavity 20. Furthermore, the device 1 advantageously comprises a plurality of detection modules 7 (made according to any one of the embodiments described above), each associated with a corresponding containment cavity 20 and with a corresponding first emitter 61.

Preferably, the detection modules 7 and the first emitters 61 are assembled on a printed circuit board and operatively connected to the logic control unit 8 to transmit the first signal S1 and the second signal S2 to the latter.

Advantageously, the first emitters 61 and the detection modules 7 may be operated jointly for performing simultaneous detections on more than one, preferably on each, containment cavity 20, or advantageously by selecting and activating one or more emitters 61 and detection modules 7 for performing detections, possibly in series, of only one or more corresponding containment cavities 20, or still advantageously by scanning with one or more emitters 61 and detection modules 7 for performing serial detections of the containment cavities 20.

In this way, it is possible to perform fast analyzes with repeatable and reproducible results on a large number of samples 3, or, alternatively, it is possible to divide the sample 3 into several containment cavities 20 to obtain several readings on the same sample 3.

Likewise, the illumination means 6 advantageously comprise a plurality of second and/or third emitters 62, 63, each associated with a corresponding containment cavity 20. Furthermore, the second and/or third emitters 62, 63 and the detection modules 7 may be operated jointly for performing simultaneous detections on each containment cavity 20, or by selection or scanning, as explained above.

According to the preferred embodiment of the invention, schematically illustrated in Figure 1, the device 1 is completely automated. Advantageously, the logic control unit 8 comprises a memory module, in which various lists of operating parameters are stored (for example average reaction time, type of sample, quantity of sample 3 and of reagent 4 to be metered) on the basis of the type of detection to be performed and biomolecule 30 to be detected. Furthermore, the device 1 comprises a control panel in data connection with the logic control unit 8 for configuring it and/or allowing the control of the device 1 by an operator.

Advantageously, the device 1 comprises several processing stations, which include a first metering station, in which the sample 3 is intended to be metered, a second metering station, in which the reagent 4 and/or the magnetic beads 41 are intended to be metered and a detection station, at which the illumination means 6 and/or at least one detection module 7 are arranged. Furthermore, the processing stations include at least one washing station, into which a washing liquid 50 is intended to be metered.

Advantageously, the device 1 comprises a first metering unit 11, which is preferably arranged at the first metering station and is adapted to automatically fill the containment cavities 20 of the container 2 with pre-established quantities of sample 3, and a second metering unit 12, which is preferably arranged at the second metering station and is adapted to fill at least one containment cavity 20 of the container 2 with a known quantity of reagent 4 and/or magnetic beads 41.

In particular, the first metering unit 11 advantageously comprises at least one first pump, for example a peristaltic pump, provided with a second delivery, and a first tube, connected to the second delivery and provided with a plurality of first branch sections, each of which ends with a corresponding first nozzle for distributing a precise quantity of sample 3 into each containment cavity 20. Advantageously, the first pump of the first metering unit 11 is in data connection with the logic control unit 8 to be actuated automatically by the latter.

The second metering unit 12 advantageously comprises at least one second pump, for example a peristaltic pump, provided with a second delivery, and a second tube, connected to the second delivery and provided with a plurality of second branch sections, each of which ends with a corresponding second nozzle to distribute a precise quantity of reagent 4 and/or magnetic beads 41 into each containment cavity 20. Advantageously, the second pump of the second metering unit 12 is in data connection with the logic control unit 8 to be actuated automatically by the latter.

Of course, without thereby departing from the scope of protection of the invention, the device 1 may comprise further metering units for each component to be introduced into the container 2, or it may comprise a single metering unit for the entire mixture to be introduced. In the latter case, the metering stations may partially or totally coincide.

Advantageously, the device 1 also comprises a washing unit 5, which is preferably arranged at the washing station and is adapted to automatically fill the containment cavities 20 of the container 2 with a predetermined quantity of washing liquid 50. Furthermore, the washing unit 5 is configured for eliminating at least part of the unreacted mixture following the introduction of the washing liquid 50.

Advantageously, the washing unit 5 comprises at least one magnet 44, which is advantageously placed in proximity to the bottom of the containment cavities 20 when the container 2 is in the washing station, as illustrated for example in Figure 1, to immobilize and retain the magnetic beads 41 within the containment cavities 20.

Of course, the magnet 44 may be conveniently positioned in any other position suitable for retaining the magnetic beads 41.

Advantageously, the device 1 preferably comprises automation means (not shown in the accompanying figures) arranged for automating detection even without the intervention of an operator.

For this purpose, the automation means comprise a transport unit, which is arranged for moving the container 2 along an operating path positioned to intercept the aforesaid processing stations and comprises for example a conveyor belt on which the container 2 is intended to be placed in support.

In particular, the conveyor belt comprises locking elements for removably fixing the container 2 to the conveyor belt itself so as to make them integral with each other.

Alternatively or in combination, the automation means optionally comprise an automated manipulator, for example a robotic arm, configured for handling the container 2.

Operatively, the components of the mixture, i.e. the sample 3, the reagent 4 and the magnetic beads 41, are initially loaded into the device 1 into the respective metering units 11, 12 and preferably the washing liquid 50, for example into respective fluid connection tanks with the corresponding metering units.

Furthermore, the container 2 is introduced into the device 1 and associated with the transport unit, for example the support on the conveyor belt.

Subsequently, the device 1 is operated, setting the desired analysis parameters by means of the control panel. Then, the container 2 is transported to the first metering station 11 to introduce the sample 3 into the containment cavities 20 of the container 2.

Advantageously, the container 2 is then transported to the detection station to carry out the detection of the qualitative state of the sample 3. If in the detection station it is determined that sample 3 has undergone haemolysis, the latter is discarded for subsequent analyses.

Subsequently, the container 2 is transported along the second metering station 12 to introduce the reagent 4 and the magnetic beads 41 into the containment cavities 20 of the container 2.

Advantageously, the container 2 is then transported back to the detection station to carry out the detection of the number of magnetic beads 41 at the first time $t_1$ by means of the first emitter 61 and the second detector 72.

Advantageously, the container 2 is then transported to the washing station where part of the unreacted mixture is removed.

Finally, the container 2 is transported back to the detection station to perform the detection by fluorescence or chemiluminescence by at least the first detector 71 and to determine again the number of magnetic beads 41 at the second time $t_2$ by the first emitter 61 and the second detector 72. In particular, the first emitter 61 and possibly the second emitter 62 irradiate the reaction cavities 20 with the respective light radiations $\lambda_e$, $\lambda_2$. If the detection is performed in fluorescence, the first emitters 61 and the second emitters 62 preferably operate at two different instants, so that the second detectors 72 and the first detectors 71 detect only the wavelength of the third light radiation $\lambda_3$ and of the first light radiation $\lambda_1$, respectively.

Therefore, the logic control unit 8 is advantageously configured for activating the first detector 71 and the second detector 72 in separate time intervals, preferably alternately over time.

Furthermore, the second emitter 62 and the first detector 71 are operated alternately to prevent the excitation radiation $\lambda_e$ from interfering with the first light radiation $\lambda_1$ emitted by the fluorophore 47 and from being detected by the first detector 71 itself.

Furthermore, if the magnetic beads 41 are provided with a fluorescent compound, the control logic unit 8 is advantageously configured for activating the first emitter 61 and the second detector 72 in separate time intervals, preferably alternately over time.

Of course, it is possible that the container 2 is brought several times to the various processing stations according to the need and the detection parameters set. It is also possible to provide several detection stations, each configured for performing a detection at different time instants during the entire analysis process.

Furthermore, without thereby departing from the scope of protection of the present invention, the device 1 may be operated manually for one or more of the operations described above, for example the introduction of the sample 3, the reagent 4 and the washing liquid 50 into the reaction cavity 20 may take place by means of an operator who meters the various components.

A further object of the invention is a method for detecting biomolecules, in particular biomolecules as defined above. Advantageously, the method in question provides for the preparation and use of a device 1 for analyzing biomolecules of the type described above and whose reference numerals will be retained for simplicity of explanation.

The method object of the invention comprises a step for preparing a container 2 provided with at least one containment cavity 20, delimiting a containment volume (preferably a plurality of containment cavities 20, each delimiting a containment volume, as explained above). In particular, the container 2 is preferably inserted by an operator inside the external structure 10 of the device 1 as described above through an access opening, so as to prevent light contamination during the detection to be performed.

The method comprises an at least partial filling step of the containment volume of the containment cavity 20 (or of at least one of the containment cavities 20) with a sample 3 susceptible of containing at least one biomolecule 30 to be detected, a reagent 4 bondable to the biomolecule 30 and susceptible of emitting a first light radiation $\lambda_1$ in fluorescence or chemiluminescence, and a plurality of magnetic beads 41. In particular, each magnetic bead 41 may be chemically bonded to the biomolecule 30, preferably through a probe molecule 42.

Advantageously, as explained above, the container 2 is moved automatically by a handling unit along an operating path which intercepts processing stations.

Advantageously, the filling step provides for a metering stage in which, in the containment cavity 20 of the container 2, the sample 3, the reagent 4 and the magnetic beads 41 are inserted.

Advantageously, in the metering stage the container 2 is filled via a first metering unit 11 with the sample 3. In particular, the metering unit 11 meters a known quantity of sample 3 within one or more containment cavities 20.

Preferably, in the metering stage the reagent 4 and/or the magnetic beads 41 are inserted into the containment cavity 20 by means of the second metering unit 12 described above.

Furthermore, the filling step advantageously comprises a reaction stage in which the biomolecule 30 to be detected in the sample 3, the reagent 4 and at least one magnetic bead 41 are bonded to each other, obtaining a discard portion of the sample 3 and/or of the reagent 4 not bonded to the magnetic beads 41. In particular, in the reaction stage part of the reagent 4 is associated with the biomolecule 30 following the hybridization of the latter with the probe molecule 42, i.e. it bonds to the probe molecule 42 and therefore to the biomolecule 30 through the latter.

Advantageously, the filling step comprises an immobilization stage of the magnetic beads 41 by applying a magnetic field in the containment cavity 20.

In particular, the immobilization stage advantageously takes place through a magnet 44, preferably placed in proximity to the bottom of the containment cavities 20, with the container 2 in the washing station.

Furthermore, the filling step advantageously comprises a washing stage, which is performed after the immobilization stage, in which a washing liquid 50 is introduced into the containment cavity 20 of the container 2 to remove the discard portion.

Advantageously, the immobilization stage ends at the same time or after the end of the washing stage, in order to retain the greatest number of magnetic beads 41 within the containment cavities 20 through the magnet 44.

Furthermore, the method comprises a step of emission of the first light radiation $\lambda_1$ by the reagent 4.

According to a first detection mode, in which the detection is performed in fluorescence, the method provides for an excitation step, in which a second emitter 62 emits an excitation radiation $\lambda_e$, which advantageously irradiates a fluorophore 47 contained in the reagent 4 and bonded to the biomolecule 30 during the reaction stage. This irradiation causes the excitation of the fluorophore 47, which advantageously emits the first light radiation $\lambda_1$.

According to a second detection mode, in which the detection is performed in chemiluminescence, the emission of the plurality of photons which make up the first light radiation $\lambda_1$ occurs spontaneously, i.e. in the absence of excitation by the excitation radiation $\lambda_e$. In particular, as explained above, to give rise to chemiluminescence, the reagent 4 is advantageously provided with a first molecule 45 susceptible of bonding to the biomolecule 30 to emit the first light radiation $\lambda_1$.

Alternatively, the molecule 45 contained in the reagent 4 is bondable to the probe molecule 42 hybridized or associated with the biomolecule 30, and susceptible of reacting with other substances of the reagent 4 to emit the first light radiation $\lambda_1$ in chemiluminescence.

For example, the reagent 4 comprises the first molecule 45 and a second molecule 46, wherein the first molecule 45 is bondable to the probe 42 hybridized or associated with the biomolecule 30, and to the second molecule 46, and possibly also with further substances of the reagent 4, to emit the first light radiation $\lambda_1$ in chemiluminescence.

The method comprises a first detection step, in which the first light radiation $\lambda_1$ is detected by means of a first detector 71, which generates a first signal S1 representative of the concentration C of biomolecule 30 within the containment cavity 20.

In particular, the first detection step may be performed several times in time to obtain different detections and the first detector 71 advantageously generates several corresponding first signals S1 at regular time intervals, for example of 1 s.

In order to determine the proportionality coefficient between the first signal S1 and the unknown concentration of the biomolecule 30 in sample 3, the method advantageously provides for a first initial calibration step, known per se in the sector and therefore not described in detail, in which the first light radiation $\lambda_1$ emitted by a series of samples 3 containing known concentrations of biomolecule 30 is detected and analyzed in order to associate a corresponding first signal S1 with each known concentration, in order to obtain a calibration straight line.

According to the idea underlying the present invention, the method further comprises an illumination step, by means of at least a first emitter 61 of a second light radiation $\lambda_2$, of the magnetic beads 41 contained within the containment volume of the containment cavity 20.

Furthermore, the above method comprises a second step of detecting a third light radiation $\lambda_3$ transmitted, reflected or emitted by the magnetic beads 41 following the absorption of at least part of the second light radiation $\lambda_2$, by means of a second detector 72, which generates a second signal S2 representative of the quantity of magnetic beads 41 within the container 2.

Advantageously, the first detection step and the second detection step are performed in separate time intervals, preferably alternately over time.

As described above, if the containment cavities 20 are advantageously arranged between the first emitter 61 and the detection module 7, the second detector 72 detects a fraction of the second light radiation $\lambda_2$ (which constitutes the third light radiation $\lambda_3$) transmitted by the first emitter 61, as the remaining part is absorbed and reflected by the magnetic beads 41.

Alternatively, if each first emitter 61 and the respective detectors 71, 72 of the detection module 7 are arranged on the same plane, above or below the containment cavities 20, the second detector 72 detects a fraction of the second light radiation $\lambda_2$ (which constitutes the third light radiation $\lambda_3$) reflected by the first emitter 61, since the remaining part is absorbed by the magnetic beads 41 and/or transmitted by the latter.

According to a further variant, in the case in which the magnetic beads 41 comprise a fluorescent compound, as anticipated above, the first emitter 61 advantageously emits the second light radiation $\lambda_2$ in fluorescence and, following the irradiation, the magnetic beads 41 (in particular their fluorescent compound) emit the third light radiation $\lambda_3$, detectable by the second detector 72. Of course, the magnetic beads 41 may also either transmit or reflect a fraction of the second light radiation $\lambda_2$, or re-emit the third light radiation $\lambda_3$, and both may be detected by the second detector 72.

Similarly to what has been described above, the method provides for a second step of initial calibration, in which the concentration of magnetic beads 41 associated with the second signal S2 is determined on the basis of a calibration straight line constructed by recording second signals S2 generated following the reception of third residual light radiations $\lambda_3$ of samples 3 containing known concentrations of magnetic beads 41.

Advantageously, if the detection is performed in fluorescence, the illumination step, in which the first emitter 61 emits the second light radiation $\lambda_2$, and the second detection step, in which the second detector 72 is active to receive the third light radiation $\lambda_3$, are performed before or after the first detection step. Therefore, in the illumination step and in the second detection step, the first detector 71 (and preferably also the second emitter 62 of the device 1 described above) is temporarily deactivated, in order to allow a detection of the third light radiation $\lambda_3$ free from interference deriving from the incidence of the first light radiation $\lambda_1$.

Furthermore, advantageously, the excitation step, in which the second emitter 62 emits the excitation radiation $\lambda_e$, and the first detection step, are alternated with each other in such a way that the first detector 71 is able to detect, in the time intervals in which the second emitter 62 is deactivated, only the first light radiation $\lambda_1$. In particular, the second emitter 62 is therefore arranged for emitting a pulsed light at regular intervals.

Irrespective of the type of detection mode of the biomolecule, if the magnetic beads 41 are provided with a fluorescent compound, preferably the illumination step, in which the first emitter 61 emits the second light radiation $\lambda_2$, and the second detection step, in which the second detector 72 is active to receive the third light radiation $\lambda_3$, are performed in separate time intervals, preferably alternately over time.

In this way, it is possible to avoid that the second detector 72 also detects the second light radiation $\lambda_2$ in addition to the third light radiation $\lambda_3$ during the second detection step.

Otherwise, if the magnetic beads 41 do not include a fluorescent compound, i.e. when the detection of the magnetic beads is performed in transmittance or absorbance mode, preferably the illumination step and the second detection step are performed simultaneously.

The method according to the invention comprises a step of calculating a correct value of concentration C of biomolecule 30 in the containment cavity 20 on the basis of at least the first signal S1 and the second signal S2.

Preferably, the calculation step is performed by the logic control unit 8 of the device 1, according to the method described above in the embodiments.

Advantageously, the illumination step and the second detection step are executed at least at a first time ti preceding the washing stage and at a second time $t_2$ after the washing stage in order to obtain at least two second signals S2', S2" representative of the quantity of magnetic beads 41 within the container 2 respectively before and after the washing stage.

Advantageously, moreover, in the calculation step, said concentration value C of biomolecule 30 is calculated based on both the second signals S2', S2".

Furthermore, the washing stage may be performed, preferably by means of the washing station 50, a number of times such as to allow the removal of most of the discard molecules, using the same washing liquid 50, or using different washing liquids 50 capable of removing unwanted molecules from sample 3, for example reaction by-products.

Preferably, each washing performed is preceded and followed by a corresponding illumination step and second detection step.

In this way, it is possible to measure the concentration of magnetic beads 41 before and after each washing and to calculate the correct concentration value C of biomolecule 30 by means of the formulas described above, i.e. for example by using the second signal S2' measured before washing as control and the second signal S2" in combination with the first signal S1 for the calculation of the concentration C.

Alternatively, it is for example possible to measure the concentration of magnetic beads 41 before and after each washing, advantageously calculating a correction parameter to correct the concentration measurement C of biomolecule 30 in sample 3 on the basis of the second signals S2', S2". This correction parameter is preferably calculated by the logic control unit 8.

Advantageously, the method also comprises a step for checking the qualitative state of sample 3, in which a third emitter 63 emits a control radiation $\lambda_c$ in the containment volume and a third detector 73 detects a fraction of control radiation $\lambda_c$ not absorbed by the sample 3 to evaluate the quality of the sample 3 itself and advantageously, to determine the presence or absence of haemolysis.

Preferably, the control step is performed after the introduction of the sample 3 into the containment cavity 20 and before the introduction of the reagent 4 and the magnetic beads 41 during the metering stage, so that the control radiation $\lambda_c$ emitted by the third emitter only irradiates the sample 3.

Of course, without thereby departing from the scope of protection of the present invention, the control step may also be performed after the introduction of the reagent 4 into the containment volume of the containment cavities 20, in particular if the risk of hemolysis of the sample 3 following the addition of reagent 4 is known.

The device and the method for detecting biomolecules thus conceived therefore achieve the intended purposes.

**Claims**

1. Device for detecting biomolecules, which comprises:

- at least one container (2) provided with at least one containment cavity (20) delimiting a containment volume intended to be at least partially filled with a mixture to be analyzed, which comprises:

- a sample (3) susceptible of containing at least one biomolecule (30) to be detected;
- a reagent (4) bondable to said biomolecule (30) and susceptible of emitting a first light radiation ($\lambda_1$) in fluorescence or chemiluminescence, and
- a plurality of magnetic beads (41), each said magnetic bead (41) being chemically bondable to said biomolecule (30);

- at least a detection module (7), associable with the containment cavity (20) of said container (2) and comprising at least one first detector (71), which is arranged for detecting said first light radiation ($\lambda_1$) coming from said containment cavity (20) in order to generate a first signal (S1) representative of the concentration (C) of biomolecule (30) within said containment cavity (20);
said device being **characterized in that** it comprises illumination means (6) comprising at least one first emitter (61) arranged for emitting at least one second light radiation ($\lambda_2$) within the containment volume of said containment cavity (20) in order to irradiate said magnetic beads (41);
said magnetic beads (41) being susceptible of at least partially absorbing said second light radiation ($\lambda_2$) and consequently transmitting, reflecting or emitting a third light radiation ($\lambda_3$) associated with the quantity of said magnetic beads (41) in said containment cavity (20);
wherein said detection module (7) comprises a second detector (72), arranged for detecting at least said third light radiation ($\lambda_3$) coming from the magnetic beads (41) contained in said containment cavities (20) and generating a second signal (S2) representative of the quantity of said magnetic beads (41) within said container (2);
said device also comprising a logic control unit (8) in data communication with said first and second detector (71, 72) and configured for:

- receiving said first signal (S1) and said second signal (S2),
- calculating, on the basis of said first signal (S1) and said second signal (S2), a correct value of concentration (C) of biomolecule (30) within said containment cavity (20).

2. Device for detecting biomolecules according to claim 1, **characterized in that** said detection module (7) comprises an integrated circuit (75) and **in that** said first detector (71) and said second detector (72) comprise, respectively, at least one first sensor (710) and at least one second sensor (720); wherein said at least one first sensor (710) and said at least one second sensor (720) are mounted on the same said integrated circuit (75).

3. Device for detecting biomolecules according to claim 1 or 2, **characterized in that** said first detector (71) comprises at least a first optical filter (74') provided with a first spectral band corresponding to said first light radiation ($\lambda_1$), and said second detector (72) comprises at least a second optical filter (74") provided with a second spectral band corresponding to said third light radiation ($\lambda_3$) and different from said first spectral band.

4. Device for detecting biomolecules according to claims 2 and 3, **characterized in that** said detection module (7) comprises a support base (70) on which said integrated circuit (75) is mounted on which at least said at least one first sensor (710) and said at least one second sensor (720) are mounted;
wherein said at least one first optical filter (74') and said at least one second optical filter (74") are fixed to the support base (70) to cover, respectively, said at least one first sensor (710) and said at least one second sensor (720).

5. Device for detecting biomolecules according to claim 3 or 4, **characterized in that** said detection module (7) comprises one or more optically opaque walls (76) interposed between said at least one first optical filter (74') and said at least one second optical filter (74").

6. Device for detecting biomolecules according to any one of the preceding claims, **characterized in that** said magnetic beads (41) are provided with a fluorescent compound and said logic control unit (8) is configured for activating said first emitter (61) and said second detector (72) in separate time intervals.

7. Device for detecting biomolecules according to any one of the preceding claims, **characterized in that**:

- said reagent (4) comprises a fluorophore (47) bondable to said biomolecule (30) and susceptible of emitting said first light radiation ($\lambda_1$) in fluorescence;
- said illumination means (6) comprise at least one second emitter (62), arranged for emitting at least one light

excitation radiation ($\lambda$e) within the containment volume of said containment cavity (20) in order to irradiate said fluorophore (47) and cause the emission of said first light radiation ($\lambda_1$) in fluorescence;
- said first detector (71) is arranged to detect said first light radiation ($\lambda_1$) in fluorescence, preferably light radiations in the visible spectrum.

8. Device for detecting biomolecules according to any one of the claims 1 to 6, **characterized in that**:

    - said reagent (4) comprises a first molecule (45) susceptible of reacting at least with said biomolecule (30) and optionally with a second molecule (46) bonded to the biomolecule (30) in order to emit said first light radiation ($\lambda_1$) in chemiluminescence;
    - said first detector (71) is adapted to detect said first light radiation ($\lambda_1$) in chemiluminescence, preferably NUV-visible light radiation.

9. Device for detecting biomolecules according to any one of the preceding claims, **characterized in that** it comprises at least one temperature sensor (9), associable with at least one between one said container (2) and said detection module (7) in order to detect a temperature value and generate at least one temperature signal relative to said temperature value;
said logic control unit (8) being configured for receiving said temperature signal and verifying that the measurement occurs in conditions of stability and correct operation of said detection module.

10. Device for detecting biomolecules according to any one of the preceding claims, **characterized in that** said container (2) is removably placed with its own containment cavity (20) interposed between the first emitter (61) of said illumination means (6) and said detection module (7).

11. Device for detecting biomolecules according to any one of the claims 1 to 9, **characterized in that** the first emitter (61) of said illumination means (6) is integrated in said detection module (7).

12. Device for detecting biomolecules according to any one of the preceding claims, **characterized in that**:

    - said container (2) comprises a plurality of containment cavities (20);
    - said illumination means (6) comprise a plurality of first emitters (61), each associated with a corresponding said containment cavity (20);
    - said device comprises a plurality of said detection modules (7), each associated with a corresponding said containment cavity (20) and with a corresponding said first emitter (61);

    said first emitters (61) and said detection modules (7) being jointly actuatable in order to execute simultaneous detections on more than one containment cavity (20).

13. Method for detecting biomolecules by means of a device according to any one of the preceding claims, said method comprising:

    - a step of arranging at least one container (2) provided with at least one containment cavity (20) delimiting a containment volume;
    - a step of at least partially filling the containment volume of said containment cavity (20) with:

        - a sample (3) susceptible of containing at least one biomolecule (30) to be detected;
        - a reagent (4) bondable to said biomolecule (30) and susceptible of emitting a first light radiation ($\lambda_1$) in fluorescence or chemiluminescence, and
        - a plurality of magnetic beads (41), each said magnetic bead (41) being chemically bondable to said biomolecule (30);

    - a step of emitting said first light radiation ($\lambda_1$) by said reagent (4);
    - a first detection step, in which said first light radiation ($\lambda_1$) is detected by means of a first detector (71), which generates a first signal (S 1) representative of the concentration (C) of biomolecule (30) within said containment cavity (20);

    said method being **characterized in that** it further comprises:

- a step of illuminating, by means of at least one first emitter (61) of a second light radiation ($\lambda_2$), said magnetic beads (41) contained within the containment volume of said containment cavity (20);
- a second step of detecting a third light radiation ($\lambda_3$) transmitted, reflected or emitted by said magnetic beads (41) following the absorption of at least part of said second light radiation ($\lambda_2$), by means of a second detector (72), which generates a second signal (S2) representative of the quantity of said magnetic beads (41) within said container (2);
- a step of calculating a correct value of concentration (C) of biomolecule (30) in said containment cavity (20) on the basis of at least said first signal (S1) and said second signal (S2).

14. Method according to claim 13, **characterized in that** said filling step provides for:

- a metering stage in which, in the containment cavity (20) of said container (2), said sample (3), said reagent (4) and said magnetic beads (41) are inserted;
- a reaction stage in which the biomolecule (30) to be detected in said sample (3), said reagent (4) and at least one said magnetic bead (41) are bonded to each other, obtaining a discard portion of said sample (3) and/or of said reagent (4) not bonded to said magnetic beads (41);
- a stage of immobilizing said magnetic beads (41) by means of the application of a magnetic field in said containment cavity (20);
- a washing stage, in which in the containment cavity (20) of said container (2), a washing liquid (50) is introduced in order to remove said discard portion;
said illumination step and said second detection step being executed at least at a first time (ti) preceding said washing stage and at a second time (tz) after said washing stage in order to obtain at least two second signals (S2', S2") representative of the quantity of said magnetic beads (41) within said container (2) respectively before and after said washing stage;
in said calculation step, said concentration value (C) of biomolecule (30) being calculated based on both said second signals (S2', S2").

15. Method according to claim 13 o 14, **characterized in that** said magnetic beads (41) are provided with a fluorescent compound and said illumination step, in which said first emitter (61) emits said second light radiation ($\lambda_2$), and said second detection step, in which said second detector (72) is active to receive said third light radiation ($\lambda_3$), are performed in separate time intervals.

Fig. 1

Fig. 2

**Fig. 3A**

**Fig. 3B**

**Fig. 4**

Fig. 5A

Fig. 5B

**Fig. 6**

**Fig. 7**

## EUROPEAN SEARCH REPORT

Application Number

EP 23 17 3727

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2014/145581 A1 (GPB DEBT HOLDINGS II LLC) 18 September 2014 (2014-09-18) * paragraph [0019] - paragraph [0037] * ----- | 1-15 | INV. G01N21/64 C12Q1/6818 C12Q1/6825 |
| Y | BEDNAR RILEY M. ET AL: "Immobilization of Proteins with Controlled Load and Orientation", APPLIED MATERIALS & INTERFACES, vol. 11, no. 40, 17 September 2019 (2019-09-17), pages 36391-36398, XP055939670, US ISSN: 1944-8244, DOI: 10.1021/acsami.9b12746 * figure 2 * & Bednar Riley M ET AL: "S-1 Supporting Information for Immobilization of Proteins with Controlled Load and Orientation This PDF file includes: Materials and Methods Supplementary Text Schemes S1-3 Figures S1-24 Tables S1-4 References 54-90", , 9 October 2019 (2019-10-09), XP093009844, DOI: 10.1021/acsami.9b12746 Retrieved from the Internet: URL:https://pubs.acs.org/doi/abs/10.1021/acsami.9b12746 [retrieved on 2022-12-20] * figure S3 * ----- | 1-15 | |

TECHNICAL FIELDS SEARCHED (IPC)

G01N
C12Q

-/--

The present search report has been drawn up for all claims

| Place of search | Date of completion of the search | Examiner |
|---|---|---|
| The Hague | 2 October 2023 | Gabriels, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## EUROPEAN SEARCH REPORT

Application Number

EP 23 17 3727

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | ZORDAN MICHAEL D ET AL: "Detection of pathogenic E. coli O157:H7 by a hybrid microfluidic SPR and molecular imaging cytometry device", CYTOMETRY A, WILEY-LISS, vol. 75, no. 2, 1 February 2009 (2009-02-01), pages 155-162, XP009115487, ISSN: 1552-4922, DOI: 10.1002/CYTO.A.20692 [retrieved on 2008-12-05] * the whole document * | 1-15 | |
| Y | US 10 900 078 B2 (ULTIMA GENOMICS INC [US]) 26 January 2021 (2021-01-26) * figure 9 * | 1-15 | |
| Y | US 5 939 021 A (HANSEN W PETER [US] ET AL) 17 August 1999 (1999-08-17) * column 3 – column 4 * | 1-15 | |

TECHNICAL FIELDS SEARCHED (IPC)

The present search report has been drawn up for all claims

| Place of search | Date of completion of the search | Examiner |
|---|---|---|
| The Hague | 2 October 2023 | Gabriels, Jan |

EPO FORM 1503 03.82 (P04C01)

page 2 of 2

# EP 4 279 904 A1

## ANNEX TO THE EUROPEAN SEARCH REPORT
## ON EUROPEAN PATENT APPLICATION NO.

EP 23 17 3727

02-10-2023

| Patent document cited in search report | | | Publication date | Patent family member(s) | | Publication date |
|---|---|---|---|---|---|---|
| WO 2014145581 | A1 | | 18-09-2014 | AU | 2014232782 A1 | 24-09-2015 |
| | | | | AU | 2014232882 A1 | 01-10-2015 |
| | | | | AU | 2018220044 A1 | 06-09-2018 |
| | | | | CA | 2905165 A1 | 18-09-2014 |
| | | | | CA | 2905178 A1 | 18-09-2014 |
| | | | | CN | 105308437 A | 03-02-2016 |
| | | | | CN | 105308458 A | 03-02-2016 |
| | | | | CN | 107817232 A | 20-03-2018 |
| | | | | CN | 108445242 A | 24-08-2018 |
| | | | | EP | 2972230 A1 | 20-01-2016 |
| | | | | EP | 2972361 A1 | 20-01-2016 |
| | | | | EP | 3933384 A1 | 05-01-2022 |
| | | | | HK | 1220759 A1 | 12-05-2017 |
| | | | | HK | 1220760 A1 | 12-05-2017 |
| | | | | HK | 1252561 A1 | 31-05-2019 |
| | | | | HK | 1258277 A1 | 08-11-2019 |
| | | | | JP | 6389868 B2 | 12-09-2018 |
| | | | | JP | 6553020 B2 | 31-07-2019 |
| | | | | JP | 2016517529 A | 16-06-2016 |
| | | | | JP | 2016524124 A | 12-08-2016 |
| | | | | JP | 2019049538 A | 28-03-2019 |
| | | | | US | 2014273277 A1 | 18-09-2014 |
| | | | | US | 2014274784 A1 | 18-09-2014 |
| | | | | US | 2015177145 A1 | 25-06-2015 |
| | | | | US | 2015177146 A1 | 25-06-2015 |
| | | | | US | 2015204867 A1 | 23-07-2015 |
| | | | | US | 2015204871 A1 | 23-07-2015 |
| | | | | US | 2017205410 A1 | 20-07-2017 |
| | | | | US | 2017212110 A1 | 27-07-2017 |
| | | | | US | 2017212111 A1 | 27-07-2017 |
| | | | | US | 2017370841 A1 | 28-12-2017 |
| | | | | US | 2017370928 A1 | 28-12-2017 |
| | | | | US | 2020371029 A1 | 26-11-2020 |
| | | | | US | 2021293709 A1 | 23-09-2021 |
| | | | | WO | 2014145581 A1 | 18-09-2014 |
| | | | | WO | 2014145619 A1 | 18-09-2014 |
| US 10900078 | B2 | | 26-01-2021 | US | 2020291469 A1 | 17-09-2020 |
| | | | | US | 2021139980 A1 | 13-05-2021 |
| | | | | US | 2022064727 A1 | 03-03-2022 |
| US 5939021 | A | | 17-08-1999 | AT | E225939 T1 | 15-10-2002 |
| | | | | CA | 2268617 A1 | 30-07-1998 |
| | | | | DE | 69808591 T2 | 03-07-2003 |
| | | | | EP | 0928420 A1 | 14-07-1999 |
| | | | | US | 5939021 A | 17-08-1999 |

EPO FORM P0459

page 1 of 2

## ANNEX TO THE EUROPEAN SEARCH REPORT
## ON EUROPEAN PATENT APPLICATION NO.

EP 23 17 3727

This annex lists the patent family members relating to the patent documents cited in the above-mentioned European search report.
The members are as contained in the European Patent Office EDP file on
The European Patent Office is in no way liable for these particulars which are merely given for the purpose of information.

02-10-2023

| Patent document cited in search report | Publication date | Patent family member(s) | Publication date |
|---|---|---|---|
|  |  | WO 9833070 A1 | 30-07-1998 |

EPO FORM P0459

## REFERENCES CITED IN THE DESCRIPTION

**Patent documents cited in the description**

- EP 2195459 A